# EUROPEAN PATENT APPLICATION

(11) **EP 3 919 381 A1**
(43) Date of publication of application: **08.12.2021**
(21) Application number: 19912417.3
(22) Date of filing: 26.12.2019
(51) Int. Cl.: B64C 39/02, B64D 47/02, G01N 21/27, A01G 7/00

(54) **GROWTH INFORMATION MANAGEMENT DEVICE, GROWTH INFORMATION MANAGEMENT SYSTEM, GROWTH INFORMATION MANAGEMENT DEVICE CONTROL METHOD AND GROWTH INFORMATION MANAGEMENT PROGRAM**

(30) Priority: 30.01.2019 JP 2019014117
(71) Applicant: TOPCON CORPORATION, Tokyo 174-8580 (JP)
(72) Inventor: AKIYAMA Shugo, Tokyo 174-8580 (JP)
(74) Representative: Mewburn Ellis LLP
(86) International application number: PCT/JP2019/051292
(87) International publication number: WO 2020/158290

(57) **Abstract**

[Problem] A growth information management apparatus is provided, which can accurately ascertain a growth situation of plants or the like regardless of a positional change of an equipment where the apparatus is mounted.

[Means of Solution] A growth information management apparatus 100 emits a measuring beam to a plant P and acquires growth information on the plant, based on received reflected light, with the growth information management apparatus being mounted on another equipment 1. The growth information is corrected based on change information on the irradiation direction of the measuring beam according to a positional change of the other equipment.

## Description

### [Technical Field]

The present invention relates to a growth information management apparatus for managing a growth situation of plants or the like in, for example, a farm field, a growth information management system, a method for controlling the growth information management apparatus, and a growth information management program.

### [Background Art]

An apparatuses for collecting data on the growth situations of plants in a farm field for growing plants such as crops has been conventionally proposed (for example, PTL 1) .

Such an apparatus is configured to emit a measuring beam to plants or the like in a farm field, receive reflected light of the beam, and ascertain the growth situation of the plants by analyzing the received reflected light.

### [Citation List]

### [Patent Literature]

[PTL 1] Japanese Patent Application Publication No. 2016-223971
[PTL 2] Japanese Patent Application Publication No. 2017-184640
[PTL 3] Japanese Patent No. 5522913

### [Summary of Invention]

### [Technical Problem]

Such an apparatus, however, emits a measuring beam while being mounted on a tractor or the like and thus cannot accurately emit a measuring beam to plants or the like due to positional changes including an inclination or a displacement of equipment such as a tractor where the device is mounted. Hence, the apparatus may fail to ascertain the growth situation of plants.

An object of the present invention is to provide a growth information management apparatus, a growth information management system, a method for controlling the growth information management apparatus, and a growth information management program, by which a growth situation of plants or the like can be accurately ascertained regardless of a positional change of equipment where the apparatus is mounted.

### [Solution to Problem]

According to the present invention, the object is attained by a growth information management apparatus emitting a measuring beam to plants and acquiring growth information on the plants, based on received reflected light, with the growth information management apparatus being mounted on another equipment, and characterized in that the growth information is corrected based on change information on the irradiation direction of the measuring beam according to a positional change of the other equipment.

With this configuration, even when the irradiation direction of a measuring beam of the growth information management apparatus (e.g., a laser growth-sensor apparatus) changes according to a positional change (e.g., an inclination or a displacement) of another equipment (e.g., a UAV) and, thus, the irradiation direction deviates from a target region or the like, a growth situation of plants or the like can be accurately ascertained by correcting the growth information.

Hence, the growth information management apparatus according to the present invention can accurately ascertain a growth situation of plants or the like regardless of a positional change of equipment where the apparatus is mounted.

The present invention is preferably characterized in that the positional change of the other equipment is detected by a tilt measuring unit and/or a position information acquisition unit.

With this configuration, the positional change of the other equipment can be detected by a tilt measuring unit (e.g., a clinometer) and/or a position information acquisition unit (e.g., a GPS device). Thus, the change can be accurately ascertained even if the other equipment is inclined or the other equipment itself is displaced or the like.

The present invention is preferably characterized in that the growth information generated based on the received reflected light is discarded when the change information on the irradiation direction of the measuring beam deviates from change information in a range of a predetermined region.

With this configuration, the growth information generated is discarded when the change information on the irradiation direction of the measuring beam deviates from change information in the range of the predetermined region. Hence, ascertaining a growth situation of plants or the like based on erroneous growth information is prevented.

The present invention is preferably characterized in that the discarded growth information is estimated based on the growth information in the vicinity thereof.

With this configuration, the discarded growth information is estimated based on the growth information in the vicinity thereof. This makes it possible to estimate more correct growth information and complement the discarded information, thereby accurately obtaining growth information on the overall farm field or the like that is an acquisition target of growth information.

The present invention is preferably characterized by including the equipment and the growth information management apparatus, the growth information management apparatus including an information collection device mounted on the equipment, and a growth information generation device for generating the growth information based on information received from the information collection device.

According to the present invention, the object is attained by a method for controlling the growth information management apparatus that emits a measuring beam to plants and acquires growth information on the plants based on received reflected light while being mounted on another equipment, the method being characterized in that the growth information is corrected based on change information on the irradiation direction of the measuring beam according to a positional change of the equipment.

According to the present invention, the object is attained by a growth information management program that causes a growth information management apparatus to perform the function of correcting growth information, the growth information management apparatus emitting a measuring beam to a plant and acquiring the growth information on the plant based on received reflected light while being mounted on another equipment, the growth information being corrected based on change information on an irradiation direction of the measuring beam according to a positional change of the equipment.

### [Advantageous Effects of Invention]

The present invention can advantageously provide a growth information management apparatus, a growth information management system, a method for controlling the growth information management apparatus, and a growth information management program, by which a growth situation of plants or the like can be accurately ascertained regardless of a positional change of equipment where the apparatus is mounted.

### [Brief Description of Drawings]

[Fig. 1]

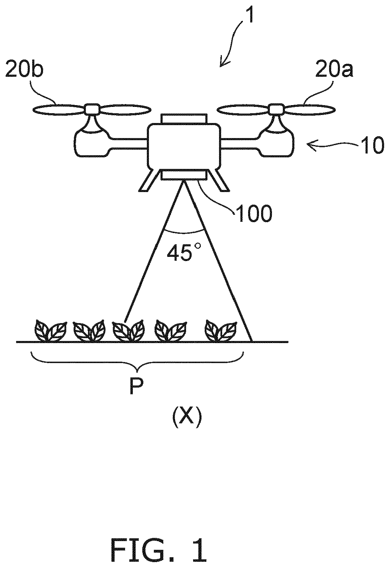

   Fig. 1 is a schematic diagram illustrating a growth information management apparatus of the present invention, for example, a laser growth-sensor apparatus mounted on another equipment, for example, a UAV (Unmanned aerial vehicle) that is also referred to as a drone.
[Fig. 2]

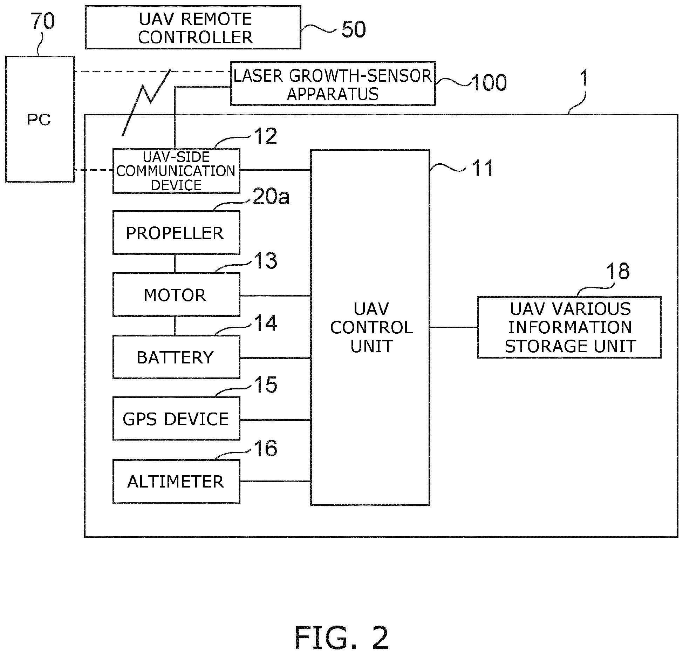

   Fig. 2 is a schematic block diagram illustrating the main configuration of the UAV including the laser growth-sensor apparatus of Fig. 1.
[Fig. 3]

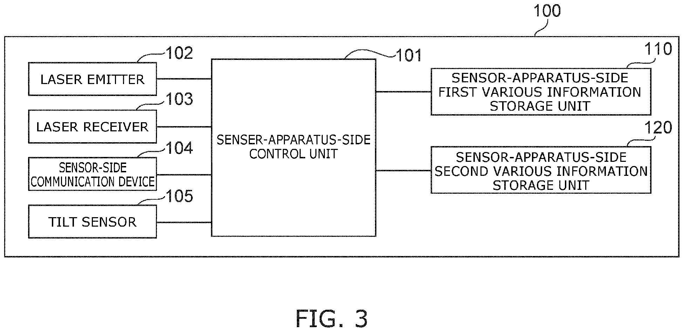

   Fig. 3 is a schematic block diagram illustrating the main configuration of the laser growth-sensor apparatus of Fig. 1.
[Fig. 4]

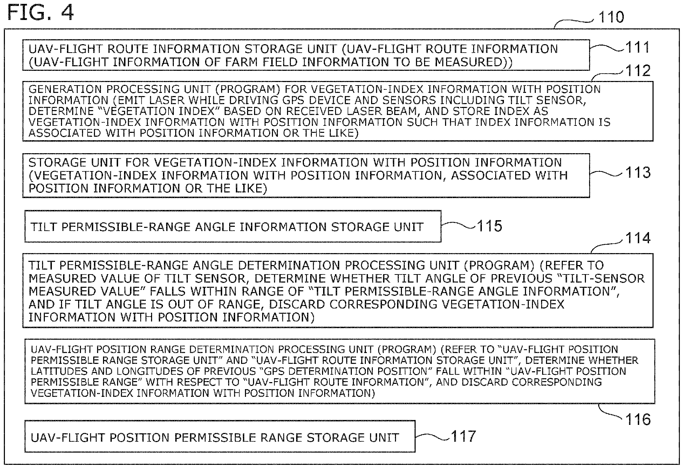

   Fig. 4 is a schematic block diagram illustrating the main configuration of a sensor-apparatus-side first various information storage unit.
[Fig. 5]

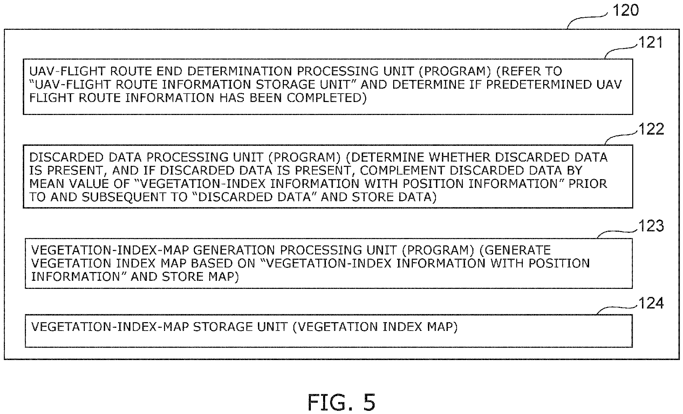

   Fig. 5 is a schematic block diagram illustrating the main configuration of a sensor-apparatus-side second various information storage unit.
[Fig. 6]

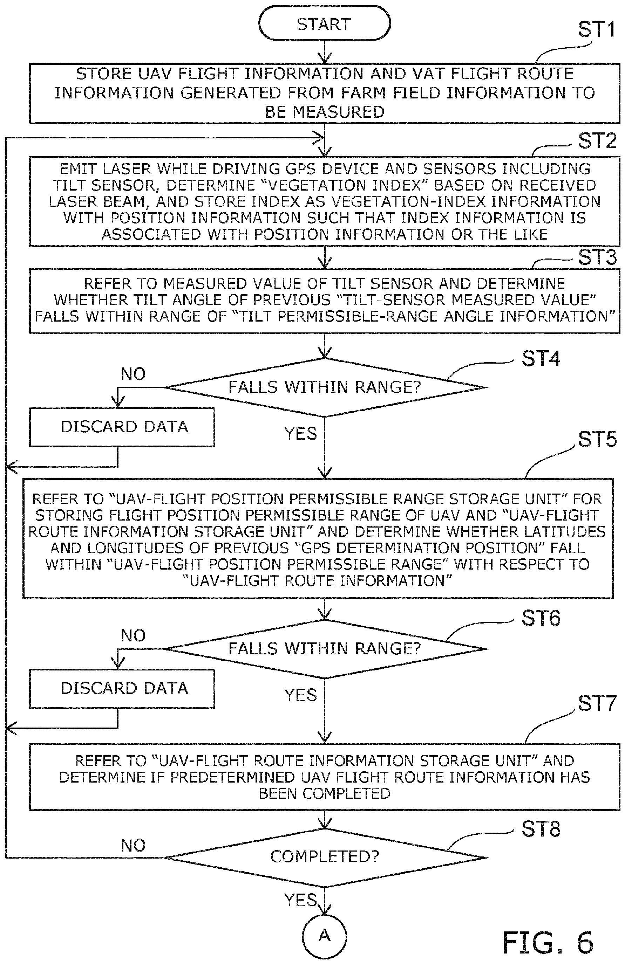

   Fig. 6 is a schematic flowchart of the steps of acquiring information on a growth situation of a plant in a farm field by using, for example, the UAV and the laser growth-sensor apparatus according to the present embodiment.
[Fig. 7]

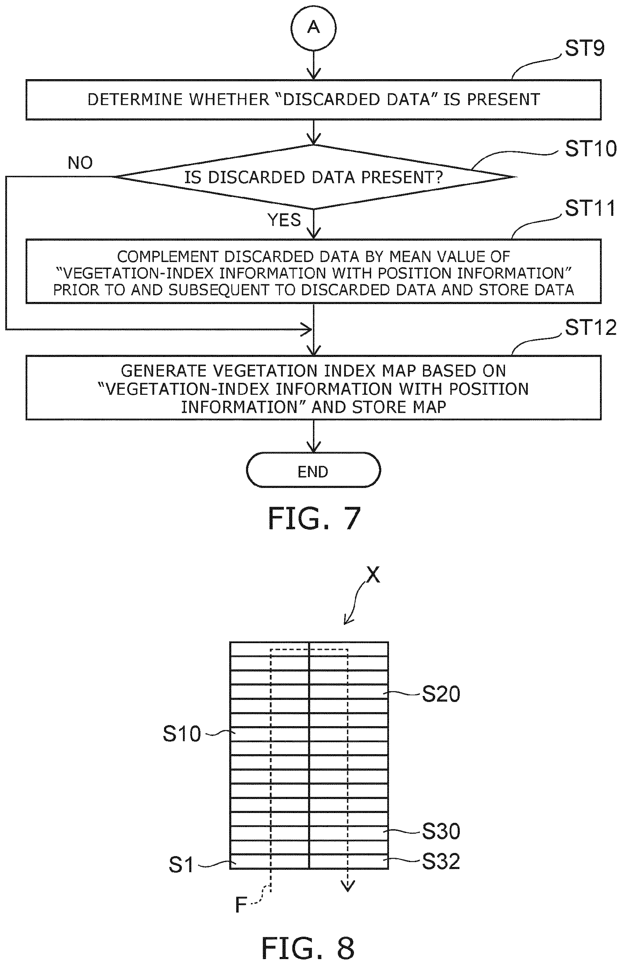

   Fig. 7 is a schematic flowchart of the steps of acquiring the information on a growth situation of the plant in the farm field by using, for example, the UAV and the laser growth-sensor apparatus according to the present embodiment.
[Fig. 8]
   Fig. 8 is a schematic diagram illustrating the farm field and the flight route of the UAV.
[Fig. 9]

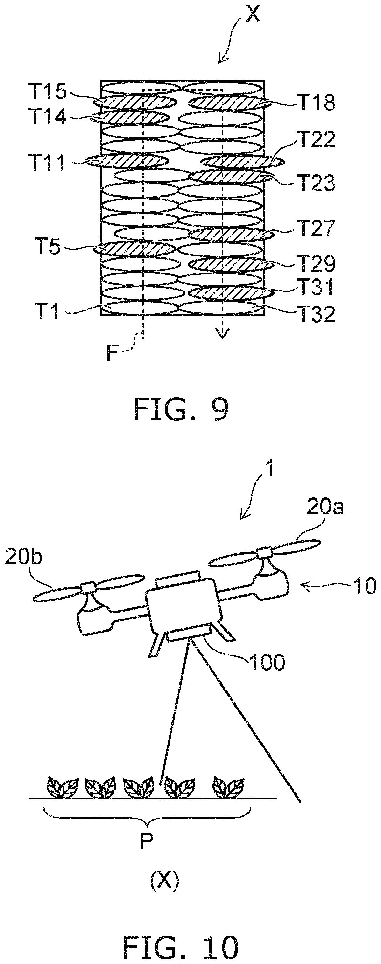

   Fig. 9 is a schematic explanatory drawing of vegetation index information (e.g., T1) actually obtained each time by the laser growth-sensor apparatus.
[Fig. 10]
   Fig. 10 is a schematic diagram illustrating a tilted attitude of the UAV.
[Fig. 11]

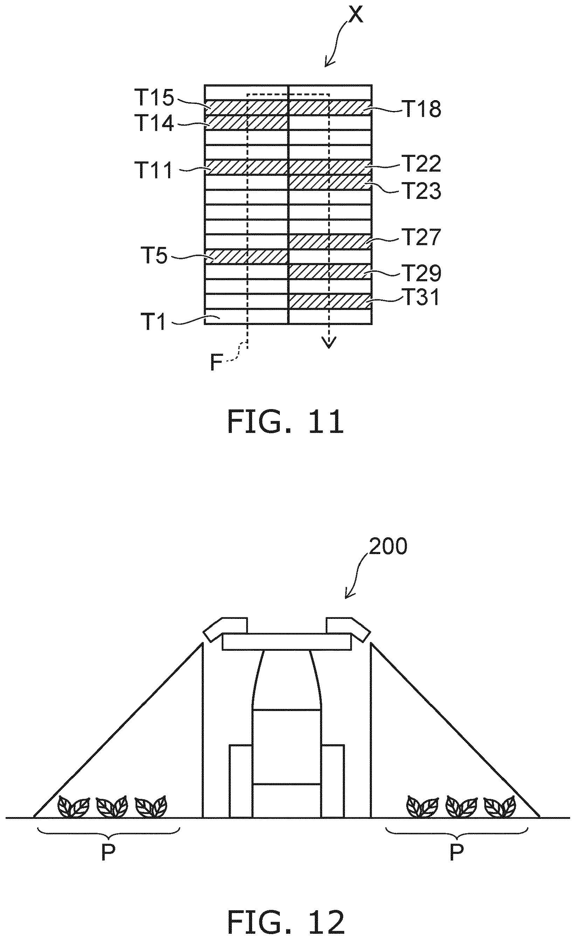

   Fig. 11 is a schematic explanation drawing indicating vegetation-index information with position information in a storage unit for vegetation-index information with position information.
[Fig. 12]
   Fig. 12 is a schematic diagram illustrating another embodiment of the present invention.

### [Description of Embodiments]

A preferred embodiment of the present invention will be specifically described below with reference to the accompanying drawings.

The following embodiment is a preferred specific example of the present invention and thus is technically limited in preferred ways. The scope of the present invention is not limited to these modes unless the present invention is limited in the following description.

Fig. 1 is a schematic diagram illustrating a growth information management apparatus of the present invention, for example, a laser growth-sensor apparatus 100 mounted on another equipment, for example, a UAV (Unmanned aerial vehicle) 1 that is also referred to as a drone.

### (Main configuration of the UAV 1)

First, the main configuration of "UAV 1" in Fig. 1 will be described below.

As illustrated in Fig. 1, the UAV 1 includes a UAV main unit 10 and multiple, for example, four propellers 20a and 20b.

In other words, the UAV 1 is configured to float with the propellers 20a and 20b or the like.

Fig. 2 is a schematic block diagram illustrating the main configuration of the UAV 1 including the laser growth-sensor apparatus 100 of Fig. 1.

As indicated in Fig. 2, in the present embodiment, "UAV remote controller 50" is provided for transmitting instruction information or the like to, for example, the UAV 1 and the laser growth-sensor apparatus 100.

The UAV remote controller 50 is configured to be operable by an operator on the ground.

Moreover, the laser growth-sensor apparatus 100 is configured to communicate with the UAV 1.

As illustrated in Fig. 2, the UAV 1 includes "UAV control unit 11." The UAV control unit 11 is configured to control, for example, "UAV-side communication device 12," "motor 13," "battery 14," "GPS device 15," "altimeter," and "UAV-side various information storage unit 18" in Fig. 2.

The configurations will be described below.

The communication device 12 is a device for communications with the UAV remote controller 50 and the laser growth-sensor apparatus 100.

In response to power supply from the battery 14, the motor 13 is configured to rotate the propellers 20a that are also illustrated in Fig. 1.

The GPS device 15 is also called "global positioning system" that three-dimensionally measures the positions of radio receivers on the earth based on the arrival times of radio waves of time signals transmitted by 24 satellites.

With this configuration, the positioning by the GPS device 15 can accurately ascertain the position (including the latitude and longitude) of the UAV 1.

The GPS device 15 is an example of a position information acquisition unit.

"Altimeter 16" is, for example, a radio altimeter that measures the reflex time of radio waves from an object, measures a distance, and measures the absolute altitude of the UAV 1.

The altimeter 16 is not limited to a radio altimeter and may be configured to measure an altitude according to a change in the atmospheric pressure of "atmospheric pressure sensor."

A tilt sensor 17 is an example of a tilt measuring unit.

"UAV-side various information storage unit 18" is a storage unit for storing a variety of information used by the UAV 1.

The UAV 1 further includes various other sensors.

For example, the UAV 1 further includes "ultrasonic sensor" acting as a sensor for detecting obstacles around the sensor, "atmospheric pressure sensor" for detecting a flying speed (detecting a speed from an atmospheric pressure), "magnetometric sensor" for measuring a bearing (compass), and "acceleration sensor" for mainly acquiring the velocity and displacement (a change of the position) of the airframe.

### (Main configuration of the laser growth-sensor apparatus 100)

Fig. 3 is a schematic block diagram illustrating the main configuration of the laser growth-sensor apparatus 100 of Fig. 1.

As illustrated in Fig. 3, the laser growth-sensor apparatus 100 includes a senser-apparatus-side control unit 101 that is configured to control "laser emitter 102," "laser receiver 103," "sensor-side communication device 104," and "tilt sensor 105."

"Laser emitter 102" in Fig. 3 has "first measuring beam" and "second measuring beam" that are lasers having different wavelengths. The first measuring beam is, for example, a beam of a red wavelength range while the second measuring beam is, for example, a beam of an infrared wavelength range.

The first measuring beam and the second measuring beam are emitted to the same plant P as illustrated in Fig. 1.

The reflected light is then received by "laser receiver 103" of Fig. 3 and the reflectivity is obtained, so that the growth situation of the plant P, specifically, the amount of a nutrient contained in the plant P can be ascertained.

The amount of the nutrient is, for example, normalized difference vegetation index (NDVI) information (hereinafter will be referred to as "vegetation index information") that is measurement growth information serving as growth information on the plant. The normalized difference vegetation index (NDVI) information will be described later.

In the present embodiment, the growth information on the plant is measured with a laser beam. In the present invention, the growth information may be acquired by satellite photographs or a sensor capable of measuring growth situations.

"Sensor communication device 104" is a device for communications with other devices.

Moreover, "tilt sensor 105" is configured to output a sensor in proportion to the tilt angle of the laser growth-sensor apparatus 100 and use the output as a tilt angle.

The tilt sensor 105 is, for example, "pendulum type" or "float type."

"Pendulum type" detects the tilt of a case or the like relative to the weight of a pendulum by using a rotation angle sensor (e.g., a magnetoresistance element or an encoder) while "float type" detects the tilt of a liquid level of liquid according to a capacitance or the like.

As illustrated in Fig. 3, the laser growth-sensor apparatus 100 also includes "sensor-apparatus-side first various information storage unit 110" and "sensor-apparatus-side second various information storage unit 120."

Figs. 4 and 5 are schematic block diagrams illustrating the main configurations of "sensor-apparatus-side first various information storage unit 110" and "sensor-apparatus-side second various information storage unit 120." The contents of, for example, the storage unit 110 will be described later.

As illustrated in Fig. 2, in the present embodiment, a PC 70 is provided as a computer removably installed on the laser growth-sensor apparatus 100 and the UAV 1.

As will be described later, the PC 70 is installed on the laser growth-sensor apparatus 100 and the UAV 1 before the flight of the UAV 1, and configured to be removed after, for example, the input of necessary information, e.g., the start of data logs.

The UAV 1 and the laser growth-sensor apparatus 100 or the like in Fig. 1 each have a computer including a CPU (Central Processing Unit), RAM (Random Access Memory), and ROM (Read Only Memory), which are connected to one another via a bus or the like and are not illustrated in Fig. 1.

### (An operation example of the present embodiment)

In the following example of the present embodiment, a user who owns a farm field X as a field of the plant P flies the UAV 1 of Fig. 1 over the farm field X and acquires information on a growth situation of the plant P.

Figs. 6 and 7 are schematic flowcharts of the steps of acquiring information on a growth situation of the plant P in the farm field X by using the UAV 1 and the laser growth-sensor apparatus 100 according to the present embodiment. (ST1)

As illustrated in Fig. 2, the PC 70 is installed on the UAV 1 and the laser growth-sensor apparatus 100 before the flight of the UAV 1. The PC 70 is removed after, for example, the input of necessary information ,e.g., the start of data logs.

Specifically, the PC 70 stores "UAV-flight route information" that is flight information on the UAV 1. The flight information is created from information on the farm field to be measured and includes altitudes, latitudes, longitudes, and velocities. For example, the UAV-flight route information is inputted to the laser growth-sensor apparatus 100.

The process then advances to step (hereinafter will be denoted as "ST") 1 in Fig. 6. In ST 1, "UAV flight route information" acquired from the PC 70 is stored in "UAV-flight route information storage unit 111" of UAV 1 in Fig. 4.

Fig. 8 is a schematic diagram illustrating the farm field X and the flight route of the UAV 1.

In Fig. 8, an arrow F of a broken line indicates the flight route of the UAV 1 in the farm field X.

A laser from the laser emitter 102 of the laser growth-sensor apparatus 100 mounted on the UAV 1 is emitted at an irradiation angle of, for example, 45°.

Thus, the flight route (including altitudes, latitudes, longitudes, and velocities) of the UAV 1 is determined in advance such that the plant P of the farm field X lies within the irradiation angle.

Specifically, in the farm field X of Fig. 8, laser irradiation over the plant P of the farm field X is completed when the laser irradiation is performed 32 times in total. The flight route is determined by calculation so as to receive reflected light.

In Fig. 8, S1 to S32 each denote a predetermined region of laser irradiation of the UAV 1.

### (ST2)

Subsequently, the process advances to ST2. In ST2, the operator operates "UAV remote controller 50" of Fig. 2 so as to fly the UAV 1 of Fig. 1. In response to the operation, the UAV 1 starts flying according to "UAV-related route information" of "UAV-flight route information storage unit 111" of Fig. 4.

Thereafter, "generation processing unit (program) 112 for vegetation-index information with position information" of the laser growth-sensor apparatus 100 operates, the laser emitter 102 of the laser growth-sensor apparatus 100 emits a laser while the GPS device 15 of the UAV 1 and sensors including the tilt sensor 105 of the laser growth-sensor apparatus 100 are driven, and the UAV 1 determines vegetation information, for example, "vegetation index (NDVI)" based on a laser beam received by the laser receiver 103 and stores the index in "storage unit 113 for vegetation-index information with position information" of Fig. 4 as "vegetation-index information with position information."

Specifically, the laser emitter 102 emits laser beams (the first measuring beam (red) and the second measuring beam (infrared)) with two different wavelengths to the plant P at the same location; meanwhile, the laser receiver 103 obtains the reflected light of each laser beam and stores a reflectivity (a reflectivity (R) of a red laser beam, a reflectivity (IR) of an infrared laser beam).

In the laser growth-sensor apparatus 100, "vegetation-index computational expression", e.g., "vegetation index (NDVI) = (IR - R)/(IR + R)" is stored in advance.

The vegetation index indicates, for example, the amount of the nutrient of the plant P.

Thus, the laser growth-sensor apparatus 100 is configured to obtain a vegetation index by substituting the reflectivity into the computational expression.

In the present embodiment, the vegetation index is used as a method for determining the growth index of a plant. Other methods may be used to identify a growth situation of a plant.

Fig. 9 indicates data obtained by the method. Fig. 9 is a schematic explanatory drawing of the vegetation index information (e.g., T1) actually obtained each time by the laser growth-sensor apparatus 100.

When the vegetation index information of one time (e.g., T1) in Fig. 9 is actually obtained, the process advances to ST3.

At this point, the UAV 1 stores the measured value of the tilt sensor 17 and positioning information (latitudes and longitudes) or the like of the GPS device 15 when a laser is emitted to obtain the vegetation index information (e.g., T1) each time in Fig. 9.

### (ST3)

Subsequently, the process advances to ST3. In ST3, "tilt permissible-range angle determination processing unit (program) 114" in Fig. 4 operates, and it is determined whether the tilt angle of previous "tilt-sensor measured value" falls within the range of "tilt permissible-range angle information" of "tilt permissible-range angle information storage unit 115" in Fig. 4 with reference to the previous measured value of the tilt sensor 17 of the UAV 1 (e.g., T1 in Fig. 8).

In "tilt permissible-range angle information" of "tilt permissible-range angle information storage unit 115" in Fig. 4, stored information indicates angles where a laser beam from the laser emitter 102 deviates from the farm field X of Fig. 7 depending upon the tilt angle of UAV 1.

Specifically, for example, if the attitude of the UAV 1 is substantially horizontal with respect to the farm field X as illustrated in Fig. 1, the range of laser irradiation falls within the farm field X, whereas if the UAV 1 is tilted by at least a certain range as illustrated in Fig. 10, the range of laser irradiation deviates from the farm field X.

Fig. 10 is a schematic diagram illustrating a tilted attitude of the UAV 1.

As described above, even if the laser growth-sensor apparatus 100 receives reflected light of a laser emitted to the farm field X and generates the vegetation, the information may be erroneous.

Thus, the present embodiment provides information on whether the tilt range of the UAV 1 falls within a permissible range or not.

A change of the range of laser irradiation according to the tilt information of the UAV 1 is an example of "information on a change of the irradiation direction of a measuring beam according to a positional change of another equipment."

Moreover, "tilt permissible-range angle information" is an example of "change information in a predetermined region."

### (ST4)

In ST4, if it is determined that the tilt angle of previous "tilt-sensor measured value" (e.g., T1 in Fig. 9 falls within the range of "tilt permissible-range angle information" of "tilt permissible-range angle information storage unit 115" in Fig. 4, the process advances to ST5.

### (ST5)

In ST5, "UAV-flight position range determination processing unit (program) 116" in Fig. 4 operates with reference to "UAV-flight position permissible range storage unit 117" that stores the flight-position permissible range of the UAV 1 in Fig. 4 and "UAV-flight route information storage unit 111" in Fig. 4.

In "UAV-flight position permissible range storage unit 117," stored information indicates a range of deviation of the UAV 1 while a laser from the laser emitter 102 of the laser growth-sensor apparatus 100 is kept within the farm field X of Fig. 8 when the UAV 1 is deviated by wind or the like from "latitudes and longitudes" information stored in "UAV-flight route information storage unit 111" in Fig. 4.

Thus, if the UAV 1 does not fall within the UAV-flight-position permissible range information of "UAV-flight position permissible range storage unit 117," an emitted laser beam deviates from the farm field X, so that even if reflected light is received and vegetation index information is generated, the information may be erroneous.

Hence, it is determined whether the latitudes and longitudes of previous "GPS determination position" of the GPS device 15 (e.g., T1 in Fig. 9) fall within "UAV-flight position permissible range" with respect to "UAV-flight route information."

### (ST6, ST7)

If the latitudes and longitudes fall within the range in ST6, the process advances to ST7. In ST7, "UAV-flight route end determination processing unit (program) 121" in Fig. 5 operates with reference to "UAV-flight route information storage unit 111" in Fig. 4, and it is determined whether the predetermined UAV-flight route information has been completed.

### (ST8)

Since only the region of T1 in Fig. 9 is completed in the foregoing example, it is determined in ST8 that the process is to continue and return to ST2 to perform the same processing on the region of T2 in Fig. 9. These steps are repeated until the region of T32 in Fig. 9 is completed.

In the example of Fig. 9 of the present embodiment, it is determined that the regions of T5, T11, T14, T15, T18, T22, T23, T27, T29, and T31 are "out of range" in ST4 or ST6.

In this case, as indicated in Fig. 6, information acquired in these regions and stored in "storage unit 113 for vegetation-index information with position information" of Fig. 4 is "discarded."

As described above, erroneous vegetation index information based on reflected light of a laser emitted outside the farm field X is "discarded," so that reliable vegetation information is generated.

In the present embodiment, the process advances to ST9 when "vegetation index information" is generated or discarded for all the regions of T1 to T32 of Fig. 9.

### (ST9)

In ST9, "discarded data processing unit (program) 122" of Fig. 5 operates, and it is determined whether "discarded data" is included in the vegetation-index information with position information of "storage unit 113 for vegetation-index information with position information" of Fig. 4.

Fig. 11 is a schematic explanation drawing indicating the vegetation-index information with position information in "storage unit 113 for vegetation-index information with position information."

In Fig. 11, "vegetation-index information with position information" includes "discarded data." Specifically, the regions of T5, T11, T14, T15, T18, T22, T23, T27, T29, and T31 indicate "discarded data."

### (ST10, ST11)

Thus, if it is determined that "discarded data" is present in ST10, the process advances to ST11. In ST11, "discarded data processing unit (program) 122" of Fig. 5 operates, and discarded data is estimated and complemented by the mean value of "vegetation-index information with position information" prior to and subsequent to "discarded data" of the vegetation-index information with position information in "storage unit 113 for vegetation-index information with position information" of Fig. 4.

For example, the region of T5 for discarded data in Fig. 11 is complemented by the mean value or the like of vegetation index information in the regions of T4 and T6. "Discarded data" of T14 and T15 is complemented by the mean value or the like of vegetation index information in the regions of T13 and T16.

In the absence of vegetation index information prior to and subsequent to "discarded data," "discarded data" may be configured to be complemented by "vegetation-index information with position information" immediately before or after "discarded data."

"Vegetation index information" having complemented "discarded data" is stored in "storage unit 113 for vegetation-index information with position information" of Fig. 4, so that vegetation-index information with position information is obtained in the absence of discarded data.

### (ST12)

Subsequently, the process advances to ST12. In ST12, "vegetation-index-map generation processing unit (program) 123" of Fig. 5 operates, and a vegetation index map is generated based on "vegetation-index information with position information" of "storage unit 113 for vegetation-index information with position information" of Fig. 4 and is stored in "vegetation-index-map storage unit 124" of Fig. 5.

Thus, by using the information on the vegetation index map, the growth situation of the plant P of the farm field X can be accurately ascertained, allowing the determination of the amount of dressing.

In the example of the present embodiment, "tilt sensor 105" is mounted in the laser growth-sensor apparatus 100 while "GPS device 15" is mounted in the UAV 1. The present invention is not limited to this configuration. For example, the laser growth-sensor apparatus 100 may include "GPS device 15."

In the example of the present embodiment, the laser growth-sensor apparatus 100 is mounted on the UAV 1. The present invention is not limited to this configuration. For example, "laser growth-sensor apparatus 100" may be mounted on "tractor 200" of Fig. 12.

Fig. 12 is a schematic diagram illustrating another embodiment of the present invention.

In Fig. 12, "tilt sensor" or "GPS device" or the like is mounted in the tractor 200 or the laser growth-sensor apparatus 100.

Furthermore, in the example of the present embodiment, all the configurations are disposed in the UAV 1 and the laser growth-sensor apparatus 100. The present invention is not limited to this configuration. A system may be configured such that the laser growth-sensor apparatus receives reflected light of a laser or perform processing until "vegetation index information" based on reflected light, the data on the processing is transmitted to a computer (a PC, e.g., the PC 70 of Fig. 2) of an operator on the ground, and subsequent processing is performed by the PC 70 or the like.

In this case, the system is an example of "growth information management system" and the laser growth-sensor apparatus is an example of "information collection device." The PC or the like is an example of "growth information generation device."

In the present embodiment, the present invention is implemented as, but is not limited to, an apparatus. The present invention may be a program executable by a computer and may be distributed while being stored in storage media including a magnetic disk (e.g., a floppy (registered trademark) disk or a hard disk), an optical disk (CD-ROM or DVD), a magneto-optical disk (MO), and semiconductor memory.

Any storage medium capable of storing programs and readable by a computer may be used. The storage format of the storage medium is not particularly limited.

Furthermore, processing for implementing the present embodiment may be partially performed by an OS (operating system) that operates on a computer in response to an instruction of a program installed on the computer from a storage medium and MW (middleware) including database management software and network software.

Moreover, the storage medium in the present invention is not limited to a medium independent of a computer. A storage medium for storage or temporary storage of a program transmitted and downloaded through a LAN or the Internet may be used.

The computer in the present invention may be any computer that performs processing in the present embodiment based on a program stored in a storage medium. An apparatus including a personal computer or a system including multiple apparatuses connected via a network may be used.

Alternatively, the computer in the present invention is not limited to a personal computer. The present invention also includes an arithmetic processing unit included in an information processor, and a microcomputer. A computer is a general name of equipment or an apparatus that can implement the functions of the present invention by means of programs.

In the foregoing explanation, the embodiment of the present invention was described. However, the present invention is not limited to the embodiment and can be changed in various ways within the scope of the claims.

### [Reference Signs List]

1 UAV
10 UAV main unit
11 UAV control unit
12 Communication device
13 Motor
14 Battery
15 GPS device
16 Altimeter
17 Tilt sensor
18 UAV-side various information storage unit
20a, 20b Propeller
50 UAV remote controller
100 Laser growth-sensor apparatus
101 Sensor-side control unit
102 Laser emitter
103 Laser receiver
104 Display
105 Various information input unit
110 Sensor-apparatus-side first various information storage unit
111 UAV-flight route information storage unit
112 Generation processing unit (program) for vegetation-index information with position information
113 Storage unit for vegetation-index information with position information
114 Tilt permissible-range angle determination processing unit (program)
115 Tilt permissible-range angle information storage unit
116 UAV-flight position range determination processing unit (program)
117 UAV-flight position permissible range storage unit
120 Sensor-apparatus-side second various information storage unit
121 UAV-flight route end determination processing unit (program)
122 Discarded data processing unit (program)
123 Vegetation-index-map generation processing unit (program)
124 Vegetation-index-map storage unit
F Flight route
P Plant
X Farm field

## Claims

1. A growth information management apparatus emitting a measuring beam to a plant and acquiring growth information on the plant, based on received reflected light, with the growth information management apparatus being mounted on another equipment,
wherein the growth information is corrected based on change information on an irradiation direction of the measuring beam according to a positional change of the other equipment.

2. The growth information management apparatus according to claim 1, wherein the positional change of the other equipment is detected by a tilt measuring unit and/or a position information acquisition unit.

3. The growth information management apparatus according to claim 1 or 2, wherein the growth information generated based on the received reflected light is discarded when the change information on the irradiation direction of the measuring beam deviates from change information in a range of a predetermined region.

4. The growth information management apparatus according to claim 3, wherein the discarded growth information is estimated based on the growth information in the vicinity thereof.

5. A growth information management system comprising:
the other equipment; and
the growth information management apparatus according to any one of claims 1 to 4, wherein
the growth information management apparatus includes
an information collection device mounted on the other equipment, and
a growth information generation device for generating the growth information, based on information received from the information collection device.

6. A method for controlling a growth information management apparatus emitting a measuring beam to a plant and acquiring growth information on the plant, based on received reflected light, with the growth information management apparatus being mounted on another equipment,
the method comprising correcting the growth information based on change information on an irradiation direction of the measuring beam according to a positional change of the other equipment.

7. A growth information management program causing a growth information management apparatus to perform a function of correcting growth information, the growth information management apparatus emitting a measuring beam to a plant and acquiring the growth information on the plant, based on received reflected light, with the growth information management apparatus being mounted on another equipment, and the growth information being corrected based on change information on an irradiation direction of the measuring beam according to a positional change of the other equipment.
